# EUROPEAN PATENT SPECIFICATION

(11) **EP 2 435 047 B1**
(45) Date of publication and mention of the grant of the patent: **23.10.2013**
(21) Application number: 10734532.4
(22) Date of filing: 01.06.2010
(51) Int. Cl.: A61K 31/519

(54) **ANGINA TREATMENT**
BEHANDLUNG VON ANGINA
TRAITEMENT D'UNE ANGINE DE POITRINE

(30) Priority: 29.05.2009 GB 0909243
(43) Date of publication of application: 04.04.2012
(73) Proprietor: The University of Dundee, Dundee DD1 4HN (GB)
(72) Inventor: STRUTHERS, Allan, Ninewells Dundee DD1 9SY (GB)
(74) Representative: Moore, Michael Richard
(86) International application number: PCT/GB2010/050923
(87) International publication number: WO 2010/136823

(56) References cited:
- TASHKENBAEVA E N: "Clinical efficiency of allopurinol and hepa-merz in unstable angina pectoris with asymptomatic hyperuricemia" MEDITSENSKKI ZHURNAL UZBEKISTANA, no. 5, 1 January 2008 (2008-01-01), pages 20-25, XP009139318 UZ ISSN: 0025-830X
- SZIEGOLEIT W ET AL: "Multicentric therapy study with allopurinol and talinolol in comparison to placebo in patients with angina pectoris" DEUTSCHE GESUNDHEITSWESEN, vol. 36, no. 7, 1 January 1981 (1981-01-01), pages 321-325, XP009139312 VERLAG VOLK UND GESUNDHEIT, BERLIN, DE ISSN: 0012-0219
- NOMAN AWSAN ET AL: "High Dose Allopurinol is Anti-Ischaemic in Chronic Stable Angina" CIRCULATION, vol. 120, no. 18, Suppl.2, 1 November 2009 (2009-11-01), page S963, XP009139315 LIPPINCOTT WILLIAMS & WILKINS, US ISSN: 0009-7322
- PACHER P ET AL: "Therapeutic effects of xanthine oxidase inhibitors: Renaissance half a century after the discovery of allopurinol" PHARMACOLOGICAL REVIEWS, vol. 58, no. 1, March 2006 (2006-03), pages 87-114, XP002602841 US ISSN: 0031-6997 DOI: 10.1124/PR.58.1.6

## Description

### FIELD OF THE INVENTION

The invention relates to compositions, therapeutic regimes and methods for the treatment of angina. In particular, the invention relates to the use of allopurinol and combinations thereof for use in the treatment of stable angina pectoris and the pain associated therewith.

### BACKGROUND OF THE INVENTION

Heart disease is a broad term that encompasses a large number of different diseases affecting the heart. It is the leading cause of death in countries such as that the United States, Canada, England and Wales. Types of heart disease include coronary artery disease (also known as ischemic coronary disease), abnormal heart rhythms or arrythmias, (congestive) heart failure, heart valve disease, congenital heart disease, heart muscle disease (cardiomyopathy), pericardial disease, aorta disease and Marfan syndrome, cardiovascular disease, peripheral vascular disease, carotid disease and arthrosclerosis. Each disease can be characterised by its different causes, symptoms and treatments.

The most common of the heart diseases is coronary artery disease (or ischemic coronary disease), in which fatty deposits (atheromatous plaques) accumulate in the cells lining the wall of the coronary arteries that supply blood and oxygen to the myocardium. This process, called atherosclerosis, leads to narrowing and/or hardening of the coronary arteries and can result in ischemia (i.e. an inability to provide adequate oxygen) and damage to heart muscle. The two main symptoms and conditions of coronary artery disease are angina pectoris ("angina") and, in more severe cases, myocardial infarction (heart attack).

Angina itself can be diagnosed as one of two sub-types, i.e. stable angina and unstable angina. Stable angina is recognised as a chest discomfort or pain (and associated symptoms), which is typically precipitated by a form or physical exertion (e.g. running, walking), with minimal or non-existent symptoms at rest. Unstable angina, however, is defined as an angina that changes or increases in its intensity. In addition, it may occur at rest and it may be severe from the moment symptoms first occur.

Treatments for stable angina can be categorised either as treatment for the relief of symptoms (e.g. to alleviate chest pain), or treatment to slow disease progression and reduce future events (e.g. to prolong survival). Treatments to prolong survival and reduce the likelihood of heart attack include: *statins* (lipid / cholesterol modifiers which probably stabilise existing atheromatous plaques); *aspirin*; *ACE inhibitors* (vasodilators); and *beta-blockers* (e.g. carvedilol, propranolol, atenolol). Treatments to reduce or alleviate the symptoms of angina include: *nitrates* (e.g. nitroglycerin); *beta-blockers*; *calcium channel blockers* (calcium agonists, such as nifedipine and amlodipine); *nicorandil* or *isosorbide mononitrate* (vasodilators); *ivabradine*; and *ranolazine* (Ranexa). Carefully controlled exercise regimes can also be a useful way of treating angina and, therefore, it can be beneficial not to avoid exercising completely. Typically, it is not possible to treat angina effectively with any single medication or treatment regime and so it is common for patients to be prescribed more than one medication (e.g. 2, 3 or 4 different drugs) in order to create an effective combination therapy. Even then, treatment regimes rarely "cure" angina and so sufferers may still experience chest pain when exercising. Continued chest pain from angina after the best drug treatments may sometimes be treated by angioplasty or bypass surgery.

Therefore, there is still a need in the art for additional therapies and drugs for treating angina. In particular, it would be desirable to have a further therapeutic drug for reducing the symptoms of chest pain on exercise, so that patients can live as normal a life as possible.

Allopurinol is a purine analogue and an inhibitor of xanthine oxidase in the body. Xanthine oxidase is responsible for the successive oxidation of hypoxanthine to xanthine and xanthine to uric acid, the product of human purine metabolism. This enzyme has been linked with a variety of diseases; and over the years, therefore, allopurinol has been used in the treatment of illnesses such as gout, hyperuricemia, ischaemic reperfusion injury, kidney stones with a uric acid component and protozoal infections (e.g. Leishmaniasis). Other xanthine oxidase inhibitors, such as oxypurinol (a metabolite of allopurinol), febuxostat and carprofen are also known.

More recently, experimental model studies have also linked allopurinol with the possible treatment of heart failure (Ekelund et al. (1999) Circ. Res., 85(5): 437-445; Ukai et al. (2001) Circulation, 103(5): 750-755; Cappola et al. (2001) Circulation, 104(20): 2407-2411); in which it has been shown to improve "*mechano energetic uncoupling*" in the myocardium. So far, this phenomenon has only been demonstrated in heart failure and almost exclusively in experimental heart failure.

The present invention addresses the above-mentioned problems in the prior art by recognising that xanthine oxidase inhibitors, such as allopurinol, can be used to treat angina, in particular, by reducing or at least alleviating the symptoms of chest pain in stable angina pectoris.

### SUMMARY OF THE INVENTION

In broad terms, the present invention provides pharmaceutical compositions, therapeutic treatment regimes, methods and kits for treating angina. The compositions of the invention can be used alone or in combination with other pharmaceutical compositions. The invention treats the symptoms of stable angina pectoris by eliminating, reducing or alleviating the pain, such as the chest pain, associated with stable angina pectoris, for example, when a patient is exercising. For example, the invention prolongs the time period during which a patient can exercise before experiencing chest pain. The pharmaceutical compositions of the invention can be used in combination with other therapeutic agents, such as known angina medications, to provide an enhanced and/or synergistic treatment regime. The invention is particularly beneficial because a number of effective xanthine oxidase inhibitors are known and have already received FDA approval in the US and marketing approval in Europe for different therapeutic uses.

Accordingly, in a first aspect, the invention provides a xanthine oxidase inhibitor for use in the treatment of one or more symptoms of stable angina pectoris; wherein the symptom is pain. Thus, the invention provides a new and important use for xanthine oxidase inhibitors. Advantageously, the provision of a new therapeutic drug treatment for angina may also mean that fewer patients will require the more risky surgery options for treating angina, as may occur when the existing medications do not work effectively. The pain symptom (pressure or discomfort)may be located in the chest, neck or arm. In one particular embodiment, the xanthine oxidase inhibitor treats at least the chest pain associated with angina.

In accordance with the invention, the xanthine oxidase inhibitor may be selected from one or more of allopurinol, oxypurinol, febuxostat and carprofen or a pharmaceutically acceptable salt or solvate thereof. Alternative xanthine oxidase inhibitors that may be useful in the invention include pterin-6-aldehyde and 6-formylpterin. In another embodiment the xanthine oxidase inhibitor is a purine analogue. Suitably the xanthine oxidase inhibitor is allopurinol or oxypurinol; and most suitably the xanthine oxidase inhibitor is allopurinol. Thus, in a most suitable embodiment, allopurinol is used to treat one or more symptoms of stable angina pectoris by eliminating or alleviating the chest pain associated therewith, and/or by delaying the time to onset of chest pain in an individual taking exercise.

It can be beneficial to administer the xanthine oxidase inhibitor in combination with one or more additional therapeutic agents, such as an agent intended to prolong survival or reduce the likelihood of heart attack. Beneficially, the additional therapeutic agents may include anti-anginal agents, which are intended to reduce, eliminate or alleviate the symptoms (e.g. chest pain) of angina. For example, the xanthine oxidase inhibitor may be administered in combination with 0, 1, 2, 3, 4, 5 or more additional therapeutic and/or anti-anginal agents. In one embodiment, the compound, molecule or composition of the invention is administered in combination with one or more therapeutic agent selected from the group consisting of: statins, for example, simvastatin, atorvastatin, fluvastatin, lovastatin, pravastatin; aspirin; angiotensin converting enzyme (ACE) inhibitors; beta-blockers, for example, carvedilol, propranolol, atenolol; nitrates, for example, nitroglycerine, isosorbide mononitrate; calcium-channel blockers, for example, nifedipine, amlodipine; nicorandil; ivabradine; and ranolazine. Suitably, the additional therapeutic agent is an anti-anginal agent that treats the symptoms of angina, such as: nitrates; beta-blockers; calcium-channel blockers; nicorandil; ivabradine and ranolazine. The xanthine oxidase inhibitor of the invention and the additional therapeutic agent(s) may be administered separately, simultaneously or sequentially.

In beneficial embodiments, the xanthine oxidase inhibitor is administered in combination with one or more of: (i) a beta-blocker; (ii) a nitrate; (iii) nicorandil; (iv) a calcium-channel blocker; (v) a beta-blocker and a nitrate; (vi) a beta-blocker and nicorandil; and (vii) a beta-blocker and a calcium-channel blocker.

In this way, the beneficial effects of the xanthine oxidase inhibitor of the invention are additive or, advantageously, synergistic with the activity of the additional therapeutic agent or anti-anginal agent. In one advantageous embodiment the additional anti-anginal agent is a beta-blocker. In another embodiment the invention relates to a combination of a xanthine oxidase inhibitor, a beta-blocker and another anti-anginal agent, such as a nitrate. Low dose aspirin may be taken in addition to any of the above drug combinations to prolong survival and/or reduce the chance of suffering a heart attack. Similarly, at least one statin may be taken in addition to any of the above drug combinations to prolong survival and/or reduce the chance of suffering a heart attack.

The xanthine oxidase inhibitor of the invention may be administered in any amount that is sufficient to provide a therapeutic effect and that is a safe dosage level. In some embodiments, the amount of xanthine oxidase inhibitor is in the range of 100 mg to 1000 mg per day. The inhibitor may be taken daily at set times, prior to exercise or at onset of the symptoms of an angina attack. For prophylactic / preventative and maintenance treatment of angina the inhibitor should be taken regularly over an appropriate period of time. Suitably, the amount of xanthine oxidase inhibitor is in the range of 300 mg to 900 mg per day. More suitably the amount of xanthine oxidase inhibitor is approximately 600 mg per day. The xanthine oxidase inhibitor may be taken twice a day (i.e. BID), or beneficially once a day.

The molecule, compound or composition of the invention may be used in any appropriate treatment regime to treat one or more symptoms of stable angina pectoris, wherein the symptom is pain. One treatment regime comprises at least three consecutive cycles (or steps) of administration: in the first cycle (or step) the xanthine oxidase inhibitor is administered in an amount of 100 mg per day (e.g. for 1 week); in the second cycle (or step) the xanthine oxidase inhibitor is administered in an amount of 300 mg per day (e.g. for 1 week); and in the third cycle (or step) the xanthine oxidase inhibitor is administered in an amount of 600 mg per day. The third cycle may comprise any suitable time period. Typically the third cycle is more than one week, for example, 4 weeks or longer (e.g. a year or more). In another treatment regime the xanthine oxidase inhibitor is administered in an amount of approximately 600 mg per day for the duration of the treatment period, for as long as prescribed. In some embodiments, the xanthine oxidase inhibitor, such as allopurinol, may be administered in dosages of 300 mg (or less) at a time. Therefore, 600 mg per day may be administered as 300 mg BID, or as a single (sustained-release) dose. Of course, in some embodiments a medical practitioner may determine the prescribed dosage according to the weight of the individual being treated.

The invention is directed to treating stable angina pectoris in an animal, suitably in a mammal, and most suitably in a human.

In a second aspect of the invention, there is provided a pharmaceutical composition comprising a xanthine oxidase inhibitor and a pharmaceutically acceptable diluent or carrier for use in treating one or more symptoms of stable angina pectoris; wherein the symptom is pain.

In one form, the pharmaceutical compositions of the invention are adapted for fast or immediate release, so that they can be taken immediately prior to exercise or at the onset of symptoms of angina (such as chest pain). In another embodiment, the pharmaceutical compositions of the invention may conveniently be in the form of modified- or controlled-release dosage forms: for example, to provide a substantially steady state level of the active agent for the therapeutic duration of the dosage form. The therapeutic duration may be suitably at least approximately 12 hours or at least approximately 24 hours. Most suitably, the pharmaceutical compositions are adapted for once-a-day administration.

A third aspect of the invention provides a kit comprising a xanthine oxidase inhibitor of the first aspect of the invention, and/or a pharmaceutical composition of the second aspect of the invention; and at least one additional therapeutic agent selected from statins, for example, simvastatin, atorvastatin, fluvastatin, lovastatin, pravastatin; aspirin; ACE inhibitors; beta-blockers, for example, carvedilol, propranolol, atenolol; nitrates, for example, nitroglycerine, isosorbide mononitrate; calcium-channel blockers, for example, nifedipine, amlodipine; nicorandil; ivabradine; and ranolazine; and instructions for use in treating angina.

It will be understood that in all aspects of the invention, the molecule, compound or composition of the invention may be as defined elsewhere herein, particularly as described in relation to the first aspect of the invention. Furthermore, it will be appreciated that the pharmaceutical composition of the second aspect of the invention may be used according to any of the methods and uses described herein, such as in relation to the first aspect of the invention. In short, any features described in relation to any aspect of the invention are incorporated into any other aspect of the invention, as appropriate.

These and other uses, features and advantages of the invention should be apparent to those skilled in the art from the teachings provided herein.

### BRIEF DESCRIPTION OF THE DRAWINGS

The invention is further illustrated with reference to the following drawings in which:
Figure 1 is a graph showing the total exercise time (in seconds) from baseline (median and IQR) for patients taking a xanthine oxidase inhibitor in accordance with the invention, in comparison to placebo, analysed by Wilcoxon Signed Rank test;
Figure 2 is a graph showing the time to ST depression (in seconds) from baseline (median and IQR) for patients taking a xanthine oxidase inhibitor in accordance with the invention, in comparison to placebo, analysed by Wilcoxon Signed Rank test;
Figure 3 is a graph showing the time to chest pain symptoms (in seconds) from baseline (median and IQR) for patients taking a xanthine oxidase inhibitor in accordance with the invention, in comparison to placebo, analysed by Wilcoxon Signed Rank test.

### DETAILED DESCRIPTION OF THE INVENTION

Prior to setting forth the detailed description of the invention, a number of definitions are provided that will assist in the understanding of the invention.

All references cited herein are incorporated by reference in their entirety. Unless otherwise defined, all technical and scientific terms used herein have the same meaning as commonly understood by one of ordinary skill in the art to which this invention belongs. Where common molecular biology techniques are described it is expected that a person of skill in the art would have knowledge of such techniques, for example from standard texts such as Sambrook J. et al., (2001) Molecular Cloning: a Laboratory Manual, Cold Spring Harbor Press, Cold Spring Harbor, NY.

In the context of the present invention, the terms "*individual*", "*subject*", or "*patient*" are used interchangeably to indicate a human or other animal that is suffering from a medical condition and may be a candidate for a medical treatment or therapeutic treatment regime of the invention. As noted, an individual may be a human subject or an animal. Thus, the methods and therapeutics of the invention may be for veterinary use. Preferred animals are mammals and the preferred mammal is a human.

A "*therapeutic compound*" or "*therapeutic agent*" as used herein, is a compound that is intended to treat (either to cure, reduce or alleviate) a disease or the symptoms of a disease, and/or to provide prophylactic, preventative or maintenance therapy to reduce the incidence or likelihood of a disease or condition (such as a heart attack or angina), or its symptoms. Thus, the term encompasses a drug, pharmaceutical molecule / compound, active agent, medicament, medicine and the like. The term "*therapeutic*" is used interchangeably with "*pharmaceutical*" in the appropriate context. Therapeutics may include compositions as well as compounds and molecules. Therefore, drugs that are used with the intention of prolonging survival or reducing the risk of heart attack in angina sufferers are referred to herein as "*therapeutic agents*".

The terms "*anti-anginal*" or "*anti-anginal agent*" are used herein to denote a therapeutic agent that is intended to "*treat*" (eliminate, reduce or alleviate) angina or the symptoms of angina. Suitably the angina is angina pectoris and more suitably stable angina pectoris; and the symptoms of angina pectoris include pain, and particularly the chest pain associated with angina.

As used herein the phrase "*therapeutic treatment regime*" refers to a series of approved, predetermined or evolving medical interventions that have the underlying intention of treating, to cure or alleviate, angina or one or more of the symptoms of angina in a patient diagnosed as suffering from that disease (or medical condition). Typically, a therapeutic treatment regime involves a patient undergoing a plurality of medical interventions that are separated in time, and which may be the same or different. A therapeutic treatment regime may also involve the regular monitoring of the patient to assess an improvement or deterioration in the health of the patient over a time period. As used herein, a "*therapeutically effective amount*" of a drug or composition is an amount that mediates either alleviation, reduction or elimination of a disease or its symptoms (e.g. the chest pain associated with angina pectoris). It may also refer to an amount that is sufficient to provide prophylactic / preventative or maintenance therapy to reduce the incidence or likelihood of a disease (such as angina) or its symptoms.

The "*efficacy of the therapeutic treatment regime*" is related to the disease state of the individual in the sense that the therapeutic treatment regime is considered to be efficacious (or effective) if the progress of the disease or the symptoms of the disease are improved under that therapeutic treatment regime. In accordance with the invention, the disease state is suitably stable angina pectoris and the treatment of the invention is effective in eliminating, alleviating or at least reducing the symptom of pain, especially chest pain, thereby to increase exercise duration before onset of symptoms.

The term "*chronic*" is intended to take its usual meaning, for example, in the sense of a chronic disease, the disease is marked by long duration, or may display frequent recurrence. Likewise, the term "*acute*" takes its normal meaning of having a sudden onset, and/or sharp rise, and/or short course.

### Heart Diseases / Cardiac Pathologies

Heart disease is a general term used to describe the numerous (perhaps more that 50) different conditions that affect the heart. These conditions include: coronary heart disease (which includes atherosclerotic coronary artery disease and coronary vasospasm); heart failure; cardiomyopathy (which can be caused by extrinsic factors or intrinsic factors); cardiovascular disease; ischemic heart disease; hypertensive heart disease (including cardiac arrhythmias); atrial fibrillation; inflammatory heart disease; and valvular heart disease; each of which may encompass more than one specific disease state.

*Atherosclerosis*, the build up of fatty deposits (plaques) within the walls of arteries that restricts the flow of blood, is probably the major underlying cause of cardiovascular disease. Ultimately, the arteries may become too blocked leading to a loss of blood flow that may affect the heart itself, the brain and other parts of the body. The build up of plaques causes the artery walls to become hard and thick, so that they lose their ability to expand and contract. At this stage, blood cannot move freely through the effected arteries. In a worst case scenario, a blood clot or plaque may become lodged in an artery, leading to complete blockage. Any body tissue that is supplied with blood (oxygen and nutrients) by that artery will then begin to die. If the blocked artery supplies the heart itself, a heart attack can occur.

*Coronary artery disease* (CAD) is the most common diagnosed form of heart disease. In CAD the arteries that supply the heart with blood are narrowed due to atherosclerosis, leading to a restriction in the flow of blood to certain parts of the heart. People with this condition, particularly early on in the disease progression, may not have any symptoms. However, in more severe cases it may result in *angina pectoris* (or angina), which is defined as a chest pain. Under resting conditions, the narrowed arteries can typically still deliver enough blood to meet the heart's needs. However, when the heart requires an increased blood supply (for example, during physical exertion, excitement, exposure to cold, or digestion of a heavy meal), the maximum blood supply to the heart muscle is insufficient to meet its demands and the chest pain of angina can occur suddenly. Angina pain is usually located under the breastbone but may also be present in the neck or arms. The pain can usually be relieved by rest and/or medication. Stable angina tends to be a chronic disease that comes and goes as described above. However, angina may also be unstable and acute. Unstable angina may occur randomly at rest; may last for 10 minutes or so; and may occur with increased frequency or ferocity over time. This disease may be a forewarning of an impending heart attack.

In accordance with the invention, at least one symptom of stable angina pectoris is removed, relieved or at least alleviated by the pharmaceutical compositions and treatment regimes described herein, and the symptom relieved or alleviated is the pain of angina (e.g. in the chest and/or neck and/or arms); and advantageously it is chest pain. Most suitably the invention is directed to prolonging the time to onset of the symptoms of angina (e.g. pain in the chest, neck and/or arm).

*Heart attack* (or myocardial infarction) may occur when a blood clot in a narrowed artery blocks the flow of blood to a part of the heart muscle. In this event, the part of the heart muscle that did not receive an adequate supply of blood will start to die and, as a result, heart action can be seriously impaired. Although a heart attack is typically a sudden event, the coronary heart disease that led up to the attack may have developed over a long period of time. Symptoms of a heart attack include uncomfortable pressure, fullness, squeezing or pain in the centre of the chest, and sometimes in the arms and shoulders, sweating, dizziness, nausea, fainting and/or shortness of breath. These (or some of these) symptoms may last for 2 minutes or more. In the more serious cases, the area of dying heart muscle can upset the normal electrical activity of the heart, such that ventricular fibrillation (twitching) occurs and the heart may be unable to effectively pump blood around the body.

*Heart failure* (or congestive heart failure) is an entirely different disease to coronary artery disease and to angina. Heart failure is essentially the condition in which the heart muscles become too weak (e.g. due to prolonged high blood pressure, heart attack, and other cardiovascular diseases) to keep blood circulating normally through the body. In this event, blood flow slows and is inadequate to meet all of the body's needs. Blood returning to the heart may also become backed up, causing swelling (e.g. in the ankles and legs). Kidneys may also be affected and not work properly and so fluid may collect in the lungs. Treatments for congestive heart failure include rest, a low salt diet, and drug therapy. Sometimes the underlying cause of heart failure can be corrected by surgery, for example, by replacing defective heart valves or arteries.

Furthermore, although heart failure, CAD and angina may all cause exercise incapacity, the causes for these effects are completely different in heart failure and CAD. In heart failure, exercise incapacity is due mainly to dyspnoea consequent upon skeletal muscle under perfusion and ergoreflex induced hyperventilation; whereas in CAD, the incapacity is due to myocardial ischaemia.

The physiological and physical differences between heart diseases such as angina and heart failure are further demonstrated by the different medical interventions / treatments (e.g. that improve exercise capacity) that are currently available for each.

By way of example, heart failure may be treated with one or more of the following medications / therapeutic agents: ACE inhibitors (used to widen the arteries); beta-blockers (reduce heart work load and heart rate); aldosterone antagonists (e.g. spironolactone); defibrillators (such as digoxin and digitalis, for control of ventricular rhythm in patients with atrial fibrillation and strengthening of heart's pumping action); and diuretics. Other therapeutic agents may be added depending on the underlying cause of the heart failure, for example: aspirin (at low dose used to help avoid future heart attacks); nitrates (including nitroglycerine to reduce angina); blood cholesterol lowering medications (e.g. statins); and blood pressure lowering medications. Meanwhile, bypass surgery (coronary artery bypass graft surgery), coronary angioplasty (balloon angioplasty), valve surgery and/or resynchronisation therapy may be used where surgery / physical intervention is an option.

On the other hand, angina treatments are selected according to whether it is intended to reduce symptoms or to prolong survival. Therapeutic treatments (agents) for prolonging survival and reducing risk of heart attack include: statins (e.g. simvastatin, atorvastatin, fluvastatin, lovastatin, pravastatin); aspirin (75 to 100 mg per day); ACE inhibitors and beta-blockers. Whereas treatments (anti-anginal agents) for the symptoms of angina (e.g. chest pain) include one or more of: nitrates (e.g. nitroglycerine and isosorbide mononitrate - vasodilators or venous system to reduce cardiac preload); beta-blockers (e.g. carvedilol, propranolol, atenolol); calcium-channel blockers (such as nifedipine and amlodipine); nicorandil (potassium channel activator - dilates the venous system as opposed to the coronary arteries); ivabradine (reduces heart rate) and ranolazine.

### Angina Pectoris Treatments

The invention is particularly directed towards treatments for "*angina pectoris*", which term is used interchangeably herein with "*angina*". Angina may be "*stable*" (typically chronic) or "*unstable*" (e.g. acute). The invention relates to treating stable angina pectoris. Treatments for angina may have the goal of prolonging survival (e.g. reducing the rate of disease progression and decreasing the likelihood of more severe symptoms such as heart attack); and/or reducing the symptoms of angina (e.g. chest pain), and/or prophylactic, preventative or maintenance of the condition. Advantageously, the present invention relates to eliminating, reducing or at least alleviating one or more of the symptoms of angina, wherein the symptom is pain. In particular, the therapeutic compositions and treatments of the invention are beneficial in reducing the chest pain associated with angina, for example, so as to prolong the exercise capability of an individual suffering from angina. In addition, the therapeutic compounds, molecules and compositions of the invention may provide for prophylactic / preventative or maintenance therapy to reduce the incidence or likelihood of angina or its symptoms. Therefore, by "*treat*" it is meant eliminate, reduce or alleviate the condition referred to, or to reduce or eliminate the likelihood of the occurrence of the condition or symptoms thereof. The term "*eliminate*" takes its normal meaning. The term "*reduce*" comprises reducing the intensity of a symptom or disease, for example, the severity of the angina and/or the intensity of a symptom, such as pain in the chest is reduced in comparison to a base level. The term "*alleviate*" encompasses a delay in onset of the condition or symptom and/or a quicker regression of the condition or symptom after onset. For example, the time taken from the start of exercise to the onset of angina (e.g. the onset of a symptom such as chest pain) is increased relative to a pre-treatment base level; and/or the duration of the chest pain is reduced relative to the pre-treatment level.

The therapeutic efficacy of a treatment of the invention can be assessed in any appropriate manner. By way of example, where the treatment relates to the symptoms of angina, such as a reduction, alleviation or elimination of pain (e.g. in the chest and/or neck and/or arm), the efficacy of the treatment regime may be assessed in an exercise duration study, such as a treadmill exercise test. The time taken from the start of the exercise routine until a particular (subjective) level of chest pain appears can be compared under equivalent conditions before (baseline level) and after the treatment (or a therapeutic treatment regime). By way of example, the conditions may include the speed of travel and the duration of exercise, and may also include constant room temperature. As described in the Examples, the invention provides molecules and compositions that extend the exercise duration of a subject until symptoms of angina are detected.

In additional to reducing the pain caused by angina, the therapeutic treatments of the invention may treat / improve other conditions experienced by a patient suffering from angina, for example, one or more of: lowering of serum uric acid levels; increase in the maximum heart rate (HR) sustainable during exercise; increase in the maximum rate pressure (RRP) during exercise; and lowering of systolic blood pressure (sBP)

The treatments of the invention may be administered in a clinical or a non-clinical setting. By "*non-clinical*" it is meant that the location or treatment is not (or is not intended to be) a medical centre, hospital, clinic or medical research laboratory or centre. Moreover, by non-clinical it is meant that the presence of a clinician (e.g. a medically trained person, such as a medic, doctor, nurse, or other therapist including a veterinary practitioner) should not be required. Advantageously, the therapeutic treatments or the invention can be self-administered and, hence, can be used in non-clinical settings, as and when required (e.g. before or during exercise). In this way, the invention beneficially allows self-management of the disease.

It is known that while single agents may reduce or alleviate a condition or symptom of angina, typically a single anti-anginal agent does not eliminate the disease. It is common, therefore, for a patient suffering from angina to be treated with one or more anti-anginal agents, which act in concert to treat the symptoms of the disease. Other therapeutic agents may also be included (e.g. aspirin and/or statins) to delay the progression of the disease. For this reason, a medical practitioner may prescribe one or more therapeutic and/or anti-anginal agents to a patient over a period of time in order to assess the most efficacious treatment regime for that individual. Some individuals do not respond to or cannot tolerate certain drugs that may be used in the treatment of angina.

Accordingly, in some embodiments of the invention, a compound, molecule or compositions of the invention (e.g. a xanthine oxidase inhibitor) is used in a combination therapy with at least one additional therapeutic agent. The at least one additional therapeutic agent may be an anti-anginal agent. The additional anti-anginal agent may be one that is intended to treat the symptoms of angina. The additional therapeutic agent may be one that is intended to prolong survival and reduce the risk of heart attack.

The compound of the invention and the therapeutic agent may act additively or, advantageously, synergistically. The compound, molecule or composition of the invention and the additional therapeutic agent(s) may be administered simultaneously, sequentially or separately. This may depend on medical advice and the recommended administration regime of the additional agent. When administered simultaneously, the agents may be in the same composition or in different compositions. The molecule, compound or composition may, therefore, be administered prior to, at the same time, or after the additional agent(s).

The duration of administration of the compound, molecule or composition of the invention (and additional agent as appropriate) may be at least one week, one month, six weeks, three months, six months, a year, or for more extended periods (such as for life). Thus, in some embodiments, the therapeutic of the invention may be taken regularly such that a therapeutically effective level of the active agent (or its metabolite) is maintained in the circulating plasma of the individual. Alternatively, the compound, molecule or composition of the invention may be suitable for administration approximately 1 hour or less (e.g. immediately) before the individual (subject / patient) exercises. In other embodiments, the compound, molecule or composition of the invention may be taken at the onset of symptoms in order to reduce the duration of the condition or symptoms associated with angina.

Suitable combination therapies of the invention may comprise the use of a xanthine oxidase inhibitor, for example, allopurinol or a salt or solvate thereof, in combination with one or more of: (i) an agent that reduces heart rate and/or load, such as a beta-blocker and/or ivabradine; (ii) an agent that is a vasodilator, such as a nitrate (e.g. nitroglycerine and isosorbide mononitrate) and/or nicorandil; an agent that reduces blood cholesterol levels, such as a statin; (iv) an agent that improves the compliance of arteries, such as an ACE inhibitor; (v) an agent that is a calcium channel blocker (or "*calcium antagonist*"); (vi) aspirin (for example, at a dosage of 75 to 100 mg per day, which is considered a "*low dose"*); (vii) an agent that is used to prolong survival and reduce risk of heart attack (such as a statin, aspirin, ACE inhibitor, and/or beta-blocker); (viii) an agent that is used to treat the symptoms of angina (such as nitrates, beta-blockers, calcium-channel blockers, nicorandil, ivabradine and ranolazine); (ix) an HMG-CoA reductase inhibitor (such as a statin, including simvastatin, atorvastatin, fluvastatin, lovastatin, pravastatin, rosuvastatin); (x) diuretics, such as thiazides (e.g. hydrochlorothiazide); and (xi) angiotensin II receptor antagonists (such as losartan, candesartin etc.). It may be beneficial to take low dose aspirin and/or a statin in addition to any of the other above drug combinations in order to help prolong survival and reduce the risk of future heart attacks.

### Xanthine Oxidase (or Xanthine Oxidoreductase)

Xanthine oxidase and xanthine dehydrogenase are interconvertable forms of the same enzyme, xanthine oxidoreductase, which generates reactive oxygen species. Xanthine oxidoreductase is widely distributed throughout various organs, including liver, gut, lungs, heart, kidney, brain as well as the plasma. However, its (relative) distribution may be different in different animals. For example, there is some debate as to whether xanthine oxidase is normally found in free blood. However, under certain conditions, such as: during severe liver damage, ischemia reperfusion injury, thoracoabdominal surgery, skin burn, and intestinal ischemia injury etc., xanthine oxidase is released into the blood.

One of the primary roles of xanthine oxidase is to catalyse the oxidation of hypoxanthine to xanthine and the subsequent oxidation of xanthine to uric acid. Therefore, the inhibition of xanthine oxidase (e.g. using allopurinol) can be used to treat hyperuricemia and gout. The enzyme also plays an important role in the catabolism of purines in some animal species, including humans.

Xanthine oxidoreductase may account for cell damage by producing reactive oxygen metabolites in cells that are reoxygenated following a period of hypoxia. By way of explanation, in normal healthy tissues, XOR exists mostly in the dehydrogenase form. However, it can be readily converted into the oxidase form, which under suitable conditions is capable of using molecular oxygen as the electron acceptor to produce substantial amounts of superoxide and hydrogen peroxide.

Xanthine oxidase makes superoxide anions which reduce the bioavailability of active nitric oxide (NO) and thus inactivates a beneficial substance (nitric oxide). Hence, by blocking xanthine oxidase, allopurinol and other xanthine oxidase inhibitors indirectly increase NO bioactivity.

### Xanthine Oxidase (or Xanthine Oxidoreductase) Inhibitors

A xanthine oxidase inhibitor is any substance that inhibits the activity of xanthine oxidase (or xanthine oxidoreductase). In humans, inhibition of xanthine oxidase is known to reduce the production of uric acid. Therefore, a xanthine oxidase inhibitor may be used to treat hyperuricemia and related medical conditions including gout. Xanthine oxidase inhibitors may be of two types: either purine analogues, or another type (i.e. not analogues of purine).

Purine analogue inhibitors of xanthine oxidase include allopurinol, oxypurinol, tisopurine, pterin-6-aldehyde, 6-formylpterin and carprofen. Other types of inhibitor include febuxostat and inositols (phytic acid and myo-inositol). Another possible inhibitor of xanthine oxidase is the natural product propolis. In accordance with the invention, any of these molecules may be used as a xanthine oxidase inhibitor. Particularly useful inhibitors are the purine analogue inhibitors, and more usefully the inhibitor is selected from allopurinol and oxypurinol. A most suitable molecule for use as an inhibitor of xanthine oxidase is allopurinol. It will be appreciated that derivatives and pharmaceutically acceptable salts and solvates of any of the xanthine oxidase inhibitors may also be used in accordance with the invention.

Allopurinol (1,5-dihydro-4H-pyrazolo[3,4-d]pyrimidin-4-one) is a highly specific competitive inhibitor of xanthine oxidase. Following its conversion to oxypurinol, oxypurinol acts as a non-competitive inhibitor.

### Dosage Forms, Medicaments and Pharmaceuticals

In accordance with the invention, the xanthine oxidase inhibitors may be manufactured into medicaments or may be formulated into pharmaceutical compositions. When administered to a subject, an active agent (including inhibitors), or pharmaceutically acceptable salts or solvates thereof of the invention is suitably administered as a component of a composition that comprises a pharmaceutically acceptable vehicle.

The molecules, compounds and compositions of the invention may be administered by any convenient route, for example, methods of administration include intradermal, intramuscular, intraperitoneal, intravenous, subcutaneous, intranasal, epidural, oral, sublingual, intranasal, intravaginal, transdermal, rectally, by inhalation, or topically, to the skin. Administration can be systemic or local. Delivery systems that are known also include, for example, encapsulation in liposomes, microparticles, microcapsules, capsules, etc., and may be used to administer the compounds of the invention. Any other suitable delivery systems known in the art are also envisioned in use of the present invention. In some circumstances, the mode of administration may be left to the discretion of the medical practitioner.

Acceptable pharmaceutical vehicles can be liquids, such as water and oils, including those of petroleum, animal, vegetable or synthetic origin, such as peanut oil, soybean oil, mineral oil, sesame oil and the like. The pharmaceutical vehicles can be saline, gum acacia, gelatin, starch paste, talc, keratin, colloidal silica, urea, and the like. In addition, auxiliary, stabilising, thickening, lubricating and colouring agents may be used. When administered to a subject, the pharmaceutically acceptable vehicles are preferably sterile. Water is a particularly suitable vehicle when the compound of the invention is administered intravenously. Saline solutions and aqueous dextrose and glycerol solutions can also be employed as liquid vehicles, particularly for injectable solutions. Suitable pharmaceutical vehicles also include excipients such as starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The compositions of the invention can also contain minor amounts of wetting or emulsifying agents, or buffering agents, as desired.

The medicaments and pharmaceutical compositions of the invention can take the form of solutions, suspensions, emulsion, tablets, pills, pellets, capsules, capsules containing liquids, powders, modified-, controlled- or sustained-release formulations, suppositories, emulsions, aerosols, sprays, suspensions, or any other form suitable for use. Other examples of suitable pharmaceutical vehicles are described in Remington's Pharmaceutical Sciences, Alfonso R. Gennaro ed., Mack Publishing Co. Easton, Pa., 19th ed., 1995, see for example pages 1447-1676.

Suitably, the compounds of the invention are formulated in accordance with routine procedures as a pharmaceutical composition adapted for oral administration (more suitably for human beings). Compositions for oral delivery may be in the form of tablets, lozenges, aqueous or oily suspensions, granules, powders, emulsions, capsules, syrups, or elixirs, for example. Thus, in one embodiment, the pharmaceutically acceptable vehicle is a capsule, tablet or pill. A suitable pharmaceutical composition for use in accordance with the invention is a commercially available allopurinol tablet / pill (e.g. from Alpharma; Teva; or "Zyloric", GSK). In another embodiment, such as where a fast acting treatment is desired (e.g. if administered immediately prior to exercise or at the onset of symptoms), the inhibitor may be administered in the form of an immediate release vehicle (e.g. a fast dissolving tablet or pill), or more suitably by sublingual spray.

In one beneficial form, the composition (e.g. tablet or pill), may be formulated to provide a controlled- or modified-release of the active agent. By "*controlled-*" or "*modified-*" release it is meant a composition (or dosage form) whose drug release characteristics, including time course and/or location are designed for convenience of administration or to accomplish particular therapeutic benefits that are not offered by immediate-release compositions. For example, such formulations may be designed to provide a quick increase in the plasma concentration of the drug (e.g. to provide a therapeutic amount of the active agent in the plasma), followed by a continual release that maintains the active agent within the therapeutic range for a desired time period. During the period of continual release the drug may be released at an essentially constant rate or at a slowly declining rate - but still at a rate high enough to maintain the desired therapeutic level for the desired duration (such as until the next dose is taken), depending on the intended characteristics of the formulation. The modified- or controlled-release dosage form / pharmaceutical composition of the invention can be contrasted to immediate-release dosage forms, which typically produce large maximum / minimum plasma drug concentrations (Cₘₐₓ/Cₘᵢₙ) due to rapid absorption of the drug into the body (i.e. *in-vivo*), relative to the drug's therapeutic index (i.e. the ratio of the maximum drug concentration needed to produce and maintain a desirable pharmacological response). In an immediate-release dosage form, the drug content is typically released into the gastrointestinal tract within a short period of time (e.g. within approximately 1 hour), and plasma drug levels peak shortly after dosing. The design of immediate-release dosage forms is generally based on getting the fastest possible rate of drug release; therefore, there can sometimes be a risk of creating undesirable dose related side effects by the resulting rapid increase in drug concentration. The modified- or controlled-release dosage forms of the invention, however, may improve the therapeutic value of the active drug by reducing the ratio of the maximum / minimum plasma drug concentration (Cₘₐₓ/Cₘᵢₙ), while maintaining drug plasma levels within the therapeutic range for a prolonged period of time. Beneficially, the medicament maintains a substantially flat serum concentration curve throughout the therapeutic period. Suitably, the prolonged therapeutic period of time is up to 24 hours, such as 8 to 24 hours, to enable the subject to take the medicament at a convenient time and frequency (e.g. once or twice daily). Accordingly, a modified- or controlled-release dosage form encompasses any medication with a release profile other than immediate release (i.e. within approx. 1 hour), such as "sustained-release", "extended-release", "slow-release", "biphasic release", "prolonged-release", and "enhanced absorption" dosage forms.

The term "*sustained-release*" as used herein is defined to mean a substantially gradual rate of release of the molecule or active agent of the invention (e.g. allopurinol). For example, a sustained-release medicament may advantageously display a steady-state release profile over a prolonged period of time *in vivo*. The rate of release of the drug is controlled by features of the dosage form and the way in which it interacts with physiological or environmental conditions. Advantageously, the sustained-release dosage forms of the invention release the drug so as to maintain a therapeutically effective concentration of active agent in the plasma of an individual for at least approximately 8 hours, at least approximately 12 hours, or more. Most beneficially, the sustained release medicament of the invention maintains a therapeutically effective level of the drug, *in vivo*, for at least approximately 24 hours, such that the medicament is suitable for administration once per day. In this way, the dosage form of the invention beneficially provides a release of the xanthine oxidase inhibitor sufficient to provide a therapeutic dose after administration, and then a gradual release over an extended period of time such that the sustained-release dosage form provides therapeutic benefit over a 24-hour period. Sustained-release dosage forms may be coated with a delayed-release coat to delay disintegration and absorption in the gastrointestinal tract, followed by a sustained-release formulation of the medicament so as to provide a sustained release of active agent over an extended period of time.

Selectively permeable membranes surrounding an osmotically active driving compound are also suitable for orally administered compositions. In these dosage forms, fluid from the environment surrounding the capsule is imbibed by the driving compound, which swells to displace the agent or agent composition through an aperture. These dosage forms can provide an essentially zero order delivery profile as opposed to the spiked profiles of immediate release formulations. A time delay material such as glycerol monostearate or glycerol stearate may also be used. Oral compositions can include standard vehicles such as mannitol, lactose, starch, magnesium stearate, sodium saccharine, cellulose, magnesium carbonate, etc. Such vehicles are preferably of pharmaceutical grade. Typically, compositions for intravenous administration comprise sterile isotonic aqueous buffer. Where necessary, the compositions may also include a solubilising agent.

A "*delayed-release*" dosage form as used herein is defined to mean dosage forms that do not substantially release drug immediately following administration but at a later (predetermined) time. Thus, delayed-release dosage forms provide a time delay prior to the commencement of drug-absorption. Such dosage forms will desirably be coated with a delayed-release coat.

Modified- and controlled-release medicament profiles can be measured *in vivo* or *in vitro* by procedures well known to the person of skill in the art. For example, by measuring the solubilisation rate of the drug (i.e. the increase in dissolved drug concentration) in an appropriate liquid (solution) *in vitro*. Bioavailability may be most appropriately assessed *in vivo*, for example, by measuring the concentration of the active agent in plasma.

Orally administered compositions may contain one or more further agents, for example, sweetening agents such as fructose, aspartame or saccharin; flavouring agents such as peppermint, oil of wintergreen, or cherry; colouring agents; and preserving agents, to provide a pharmaceutically palatable preparation.

Allopurinol is only slightly soluble in water and alcohol; practically insoluble in chloroform and in ether; and it dissolves in dilute solutions of alkali hydroxides. It can be used in this manner, but alternatively, to improve its solubility in water, a salt such as the sodium salt can be used instead of the base.

In accordance with the invention, the inhibitor of xanthine oxidase may also be administered together with another pharmaceutically active agent, such as one or more (e.g. 1, 2, 3, 4, 5 or more) anti-anginal agents. The xanthine oxidase inhibitor acts synergistically or at least in an additive manner with the one or more additional anti-anginal agent, depending on the particular agent concerned.

Where the invention provides more than one active agent for use in combination, generally, the agents may be formulated separately or in a single dosage form, depending on the prescribed most suitable administration regime for each of the agents concerned.

The amount of xanthine oxidase inhibitor that will be effective in the treatment of angina may depend on the severity of the disorder, other anti-anginal agents that may be used in combination, and on the patient's specific circumstances. An appropriate amount of inhibitor may be determined using standard clinical techniques, or may be prescribed by a medical practitioner. In some cases, the precise dose to be used may also depend on the route of administration. However, suitable dosage ranges for xanthine oxidase inhibitors, and particularly for allopurinol, are from about 100 mg to about 1000 mg per day or per administration. Generally, the amount is the amount administered each day of treatment. In some embodiments, the amount of xanthine oxidase inhibitor (e.g. allopurinol) is in the range of about 300 mg to about 900 mg per day. More suitably, the amount of xanthine oxidase inhibitor (e.g. allopurinol) is in the range of about 450 mg to about 750 mg per day, or about 500 mg to about 700 mg per day. Still more suitably the amount of xanthine oxidase inhibitor (e.g. allopurinol) is approximately 600 mg per day, which may be administered at about 300 mg BID. Alternatively, allopurinol may conveniently be administered in a sustained- (or slow-) release form to allow a once daily administration of the medicament comprising the desired daily dose of the xanthine oxidase inhibitor. The required amount of the xanthine oxidase inhibitor to be administered may be determined on the basis of the weight of the patient (e.g. in the range of 2 to 20, or 5 to 10 mg per kg of body weight per day). In some cases it can be beneficial to increase the amount of xanthine oxidase inhibitor over time (e.g. to check and avoid potential side effects at first). For example, a suitable stepped treatment regime is: step 1, 100 mg per day; step 2, 300 mg per day; step 3, 600 mg per day (e.g. 300 mg BID). Typically, steps 1 and 2 are 1 week each, although any appropriate duration may also be suitable (e.g. 1 to 4 weeks). Step 3 is typically at least 1 week in duration, but may be any appropriate time while the subject is experiencing angina, for example, at least 4 weeks to life of the individual. Depending on the severity of the condition or the response to treatment, it may be beneficial to increase the highest dose of xanthine oxidase inhibitor to greater than 600 mg, for example, to approximately 900 mg. The higher dose may be used either for the entire duration of treatment; for step 3; or in a further step (step 4). These amounts are particularly suitable where the xanthine oxidase inhibitor is allopurinol. Typically, where used, the dosage of oxypurinol is comparable to that of allopurinol.

Any suitably duration of treatment may be used, for example, at least 1 week, at least 1 month, at least 6 weeks, at least 2, 3 or 6 months, at least 1 year, or longer (such as for life).

### Kits

The invention further relates to kits for use in treating angina.

A suitable kit comprises at least two therapeutic agents: (1) a xanthine oxidase inhibitor of the invention or a pharmaceutical composition of the invention; and (2) at least one additional therapeutic agent. Beneficially, the kit also includes instructions for use, for example, to direct the user (individual) to take certain of the therapeutics agents at a particular time or interval.

The at least one additional therapeutic agent may be is selected from statins, for example, simvastatin, atorvastatin, fluvastatin, lovastatin, pravastatin; aspirin; ACE inhibitors; beta-blockers, for example, carvedilol, propranolol, atenolol; nitrates, for example, nitroglycerine, isosorbide mononitrate; calcium-channel blockers, for example, nifedipine, amlodipine; nicorandil; ivabradine; and ranolazine; and instructions for use in treating angina. Advantageously, the additional therapeutic agent may be an anti-anginal agent as previously described.

A kit is advantageous for administering a combination of therapeutic agents when, for example, the components must be administered at different time intervals or when they are in different dosage forms.

### EXPERIMENTAL - MATERIALS AND METHODS

### Example 1

### 1. Methods

Unless otherwise specified, the practice of the present invention will employs conventional techniques in medicine, pharmacology and biochemistry, which are within the capabilities of a person of ordinary skill in the art.

### 1.1 Study Overview

A randomised, double-blind, placebo-controlled, cross-over study was conducted to demonstrate a therapeutic effect of allopurinol in the treatment of angina pectoris. The trial was carried out at Ninewells Hospital, UK. It was approved by the Fife, Forth Valley and Tayside Research Ethics Committee and was conducted in accordance with the Declaration of Helsinki. Signed, written informed consent was obtained from each participant.

### 1.2 Study Protocol

Individuals aged 18 to 85 years were eligible if they had angiographically documented coronary artery disease, a positive exercise tolerance test (ETT) and a history of symptoms of chronic, stable, effort-induced angina for ≥ 2 months. Concomitant antianginal medication was allowed.

Exclusion criteria included myocardial infarction ≤ 2 months, coronary revascularization (percutaneous or CABG) ≤ 6 months, Left Ventricular Ejection Fraction <45%, estimated GFR <45 ml/min, significant valvular pathology, already on allopurinol, atrial arrhythmias or ECG abnormalities interfering with ST-segment interpretation.

An initial history and clinical examination were performed. Participants then underwent an ETT using the full Bruce protocol. A second ETT was performed within 14 days. Eligible participants had to manifest ischaemia (ST depression ≥ 1 mm compared to resting ECG) on both visits with a between visit difference in time to ST depression of less than 15%. Otherwise, a third ETT was performed and there had to be a difference of less than 15% between the second and third test.

Eligible participants were then randomised to either allopurinol up to 300 mg BID or matching placebo for 6 weeks. The dose of allopurinol started at 100 mg OD for the first week, 300 mg OD for the second week and 300 mg BID for the remaining treatment period. Participants were than crossed to the other treatment for a further 6 weeks. Subjects were allowed to continue with all their existing anti-angina medications, which remained unchanged throughout. At the end of each treatment period, patients were clinically assessed and underwent a further ETT after each treatment arm.

The ECG analysis was performed by two independent observers who were both blind to the treatments. The results of each assessment were virtually identical.

### 1.3 Statistical Analysis

Tables 1 and 2 provide breakdowns of the existing anti-anginal medication regimes taken by participants in the treatment group.

As indicated in Table 1, the number of existing anti-anginal agents prescribed to participants in this study ranged from 0 to 4. Table 2 does not provide a full analysis of the existing prescribed drug combinations taken by participants in the study. As indicated, 52 patients were already taking at least a beta-blocker; 28 patients were already taking at least an oral nitrate; 13 patients were already taking at least nicorandil; 13 patients were already taking at least a calcium channel blocker; 26 patients were already taking at least a beta-blocker and a nitrate; 12 patients were already taking at least a beta-blocker and nicorandil; and 12 patients were already taking at least a beta-blocker and a calcium channel blocker.

**Table 1: Number of prescribed anti-anginal agents taken by participants.**

| **No. of anti-anginal agents** | **No. of Participants** |
|---|---|
| 0 | 4 |
| 1 | 21 |
| 2 | 22 |
| 3 | 10 |
| 4 | 3 |

**Table 2: Number of prescribed anti-anginal agents taken by participants, excluding aspirin.**

| | **None** | **Beta-blocker** | **Nitrate** | **Calcium antagonist** | **Nicorandil** | **Not this agent** |
|---|---|---|---|---|---|---|
| **None** | 4 | 2 | 2 | 1 | 1 | 56 |
| | | | | | | |
| **Beta-blocker** | 2 | | 26 | 12 | 12 | 8 |
| **Nitrate** | 2 | 26 | | | | 32 |
| | | | | | | |
| **Calcium antagonist** | 1 | 12 | | | | 47 |
| **Nicorandil** | 1 | 12 | | | | 47 |
| | | | | | | |
| **Not this agent** | 56 | 8 | 32 | 47 | 47 | |

Results are presented as mean ± SD where parametric tests were used and median and interquartile range where non-parametric tests were used. For parametric tests, comparisons between the treatments (allopurinol and placebo) and baseline were performed using repeated measures ANOVA. 2-tailed paired Student *t* test was used for comparing changes from baseline. The non-parametric tests employed were Friedman's and Wilcoxon signed rank tests. The Grizzle method was used to assess for carry over effects. A *p* value <0.05 was considered significant.

Power calculations were based originally on an expected average improvement of 50 seconds in the time to ST depression and a standard deviation of 90 seconds. Only 34 individuals were needed in a crossover study to have 90% power at p<0.05. However, in this study the sample number was increased to 60 completed individuals overall.

### 2. Results

### 2.1 Study sample

103 participants had screening ETTs. 65 participants met the ETT criteria and were randomised. The rest of the screened participants were excluded, usually as a result of a negative screening ETT. Of the 65 randomised participants, 5 withdrew before completion due to reasons unrelated to the study. The dropouts were all excluded from analysis as none of them completed any course of treatment and none had an ETT except for their baseline one. It is worth noting that no adverse effects of treatment were seen. Table 3 shows the baseline characteristics of the participants.

### 2.2 Biochemical Data

Serum analysis demonstrated that the treatment with allopurinol significantly lowered serum uric acid (baseline 0.36 ±0.06 mmol/L; placebo 0.36 ±0.08 mmol/L; allopurinol 0.14 ±0.05 mmol/l; p<0.001). There were no significant differences in haemoglobin, serum urea, creatinine, and total cholesterol.

### 2.3 Treadmill Exercise Testing

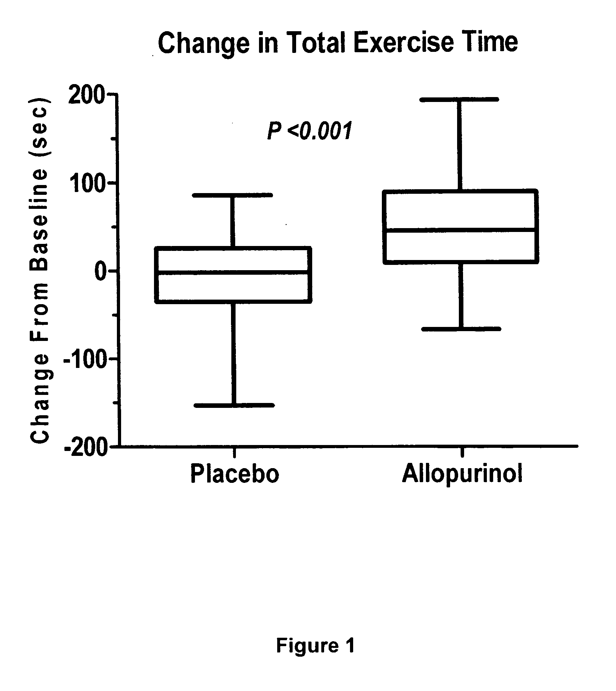

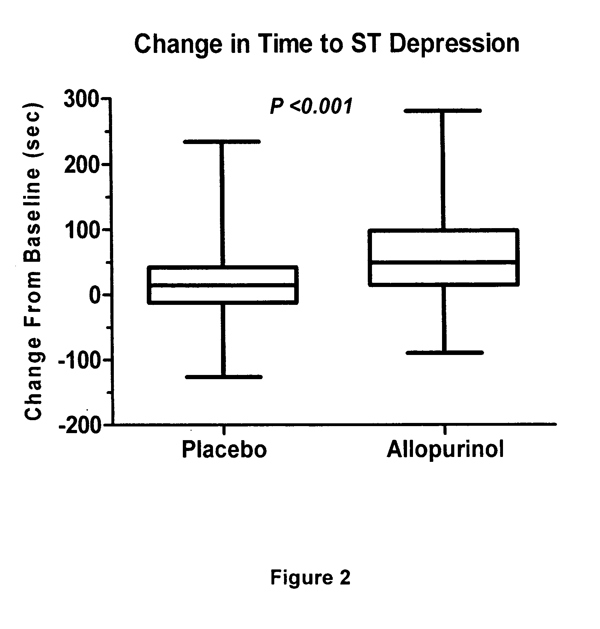

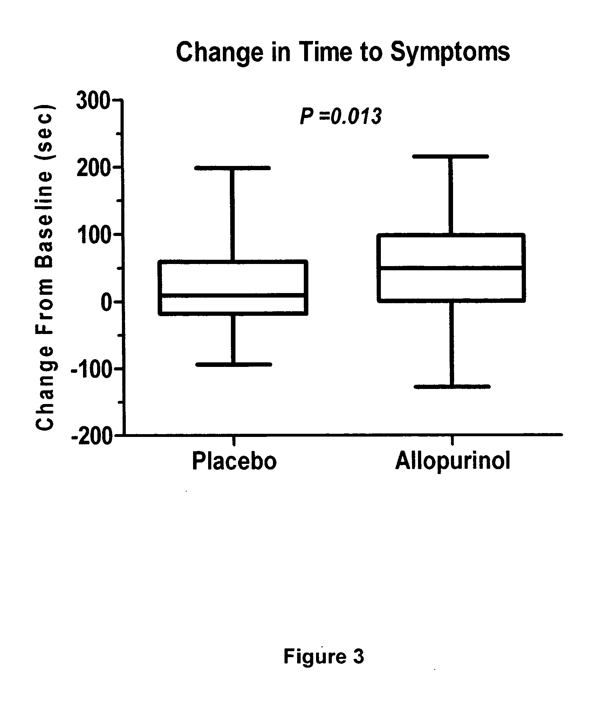

The results of the treadmill exercise tests are displayed in Table 4 graphically in Figures 1 to 3. These data are displayed as median and interquartile ranges [IQRs]. Figure 1 shows the total exercise time for patients before and after allopurinol treatment. The data demonstrates that allopurinol treatment results in a significant increase in total exercise time. Figure 2 demonstrates the time to ST depression for participants before and after allopurinol treatment. The data demonstrates that allopurinol treatment results in a significant increase in time to ST depression. Figure 3 shows the time to onset of angina symptoms for participants before and after allopurinol treatment. The data shows that allopurinol treatment results in a significant increase in time to onset of angina symptoms (e.g. chest pain). Baseline urate levels did not significantly correlate with the allopurinol induced change in time to ST depression or to symptoms. There was, however, a weak and nominally significant correlation between baseline urate and the effect of allopurinol on total exercise time (correlation co-efficient 0.27; p=0.039).

No carryover effects at all were seen for time to ST depression or time to symptoms. As indicated, the median exercise time was very similar on placebo as at baseline whereas it was 86 to 92 seconds (30%) higher on allopurinol.

### 2.4 Haemodynamic Data

This data is shown in Table 5. These data are normally distributed. The maximum heart rate (HR) and rate pressure product (RPP) during exercise was significantly higher with allopurinol treatment compared to placebo (in addition to existing medication regimes). This may be a reflection of the longer total exercise period for participants receiving allopurinol.

At the end of the first stage of the Bruce protocol, the systolic blood pressure (sPB) was significantly lower with allopurinol compared to placebo (-6.1 mmHg for allopurinol vs -2.4 mmHg for placebo; p=0.026). Without being bound by any particular theory, this may be a reflection of improved endothelial function with allopurinol, which manifested itself as a lesser increment in sBP during exercise because of improved arterial compliance during exercise.

**Table 3: Test subject statistics and relevant data.**

| **Baseline Characteristics** | | |
|---|---|---|
| | Age, y | 64.6 ±9.3 |
| | Sex (male/female), n | 50/10 |
| | Angina CCS (I/II/III), n | 9/42/9 |
| | No. CAD (1/2/3), n | 10/24/26 |
| | LVSF (normal/mild impairment) | 51/9 |
| | Renal Function (normal/ mild impairment) | 55/5 |
| Past medical history, n (%) | | |
| | Hypertension | 27 (45) |
| | Diabetes mellitus | 7 (11.7) |
| | Hypercholesterolaemia | 26 (43.3) |
| | PVD | 1 (1.7) |
| | CVA or TIA | 4 (6.7) |
| | Previous MI | 12 (20) |
| | Previous PCI | 7 (11.7) |
| | Previous CABG | 7 (11.7) |
| | Smoking (current/ex/non) | 5/ 31/ 24 |
| Medications, n (%) | | |
| | Aspirin | 60 (100) |
| | Beta-Blocker | 52 (86.7) |
| | Oral Nitrate | 29 (48.3) |
| | Calcium Antagonists | 13 (21.7) |
| | Nicorandil | 13 (21.7) |
| | ACE inhibitor | 28 (46.7) |
| | Angiotensin Blocker | 6 (10) |
| | Statin | 58 (96.7) |
| Serum urea (mmol/l) | | 6.4 ±1.5 |
| Serum creatinine (mmol/l) | | 84.9 ±16.1 |
| Serum cholesterol (mmol/l) | | 4.1 ±1 |
| LDL cholesterol (mmol/l) | | 1.9 ±1 |
| HDL cholesterol (mmol/l) | | 1.3 ±0.3 |
| Haemoglobin (g/dL) | | 13.8 ±1.3 |

**Table 4: Absolute values (median and IQR) for baseline, placebo and allopurinol in total exercise time, time to ST depression and time to symptoms.**

| | **Baseline** | **Placebo** | **Allopurinol** | **P value** |
|---|---|---|---|---|
| Total Exercise Time (secs) | 301 [251-447] | 307 [232-430] | 393 [280-519] | <0.001 |
| | | | | |
| Time to ST Depression (secs) | 232 [182-380] | 249 [200-375] | 298 [211-408] | <0.001 |
| | | | | |
| Time to Symptoms (secs) | 234 [189-382] | 272 [200-380] | 304 [222-421] | p=0.001 |

### 3. Discussion

During a standard exercise test, allopurinol significantly improved the total exercise time; the time to ST depression; and the time to angina symptoms in patients with chronic stable angina. These data suggest that endogenous xanthine oxidase activity may contribute to exercise induced myocardial ischaemia and that xanthine oxidase inhibitors are useful in treating angina. They also show that allopurinol is a novel anti-ischaemic agent in stable angina pectoris. Also the data demonstrates that allopurinol can be used to treat angina and the symptoms of angina, such as chest pain.

The magnitude of the anti-ischaemic effect seen with allopurinol is at least as good and in most cases better than that seen for other known anti-anginal agents. For example, the absolute increase in median time to ST depression with allopurinol was 66 seconds, i.e. a 28% increase (22% by geometric mean). Comparable published figures for other anti-anginal agents are 36 seconds (= 13%) for amlodipine; 60 seconds (= 11%) for nitrates, 12-47 seconds for phosphodiesterase inhibitors; 46 seconds (= 13.5%) for ivabradine; and around 50 seconds (= 15%) for atenolol and ranolazine ¹⁻⁷.

Without being bound by any particular theory, the anti-ischaemic effect and angina treatment efficacy of allopurinol might be due to its effect on myocardial energetics / oxygen consumption, but this effect could be supplemented by better coronary blood flow and reduced LV afterload. In fact, its effect on energetics and its effect on endothelial / vascular function could be interlinked since mechanoenergetic uncoupling appears to occur when the xanthine oxidase (XO) / nitric oxide synthase (NOS) balance has XO upregulated and NOS downregulated ⁸. The latter is, in fact, a common situation in cardiovascular disease and also one where xanthine oxidase inhibitors, such as allopurinol, are liable to be particularly advantageous as they can favourably alter both XO and NO. In fact, based on the data provided herein, xanthine oxidase inhibitors, such as allopurinol, may be particularly suitable as anti-anginal agents, because they may be capable of any one or more of: directly improving myocardial energetics / O₂ consumption; increasing coronary blood flow; and reducing LV loading. Allopurinol may be capable of doing all three of these.

Angina (e.g. chronic stable angina) is known to contribute to a significant reduction in the quality of life and in life expectancy, and many patients still fall short of the ACC/AHA guideline goal of complete absence of exertional angina episodes ⁹. As a result, new therapies are needed. Xanthine oxidase inhibitors can now be considered as a potential new anti-anginal agent. Furthermore, the use of xanthine oxidase inhibitors such as allopurinol has many advantages over the many other anti-anginal agents currently available. For instance, unlike anti-anginal agents, such as ranolazine and ivabradine, allopurinol is inexpensive and has a known long-term safety record. In comparison to older anti-anginal agents such as nitrates and beta-blockers, allopurinol is arguably better tolerated in that it does not reduce blood pressure or heart rate, nor does it produce many side effects, such as headaches and tiredness that occur not infrequently with nitrates and beta-blockers.

**Table 5: Haemodynamic responses during exercise testing (mean ± SD)**

| **Variables** | | **Baseline** | **Placebo** | **Allopurinol** | ***P*** |
|---|---|---|---|---|---|
| HR (bpm) | | | | | |
| | Resting | 62.3±10.3 | 61.3±9.2 | 63.8±8.6 | NS |
| | Stage 1 | 95.2±13.7 | 94.3±13.3 | 95.6±13.5 | NS |
| | Peak exercise | 113.6±15.3 | 112.4±15.6 | 118.5±15.2 | <0.001 |

| sBP (mmHg) | | | | | |
|---|---|---|---|---|---|
| | Resting | 126.8±16.6 | 124.3±13.7 | 123.7±16.2 | NS |
| | Stage 1 | 141.6±21.0 | 140.0±16.1 | 135.5±19.3 | 0.026 |
| | Peak exercise | 159.3±22.6 | 155.1±18 | 158.7±22.4 | NS |

| dBP (mmhg) | | | | | |
|---|---|---|---|---|---|
| | Resting | 72.8±8.6 | 72.9±7.7 | 72.2±9.9 | NS |
| | Stage 1 | 72.9±10.6 | 74.8±8.6 | 71.7±10.1 | NS |
| | Peak Exercise | 76.1±12.7 | 78.5±10.2 | 75.4±11.9 | NS |

| RPP (bpm.mmHg) = Rate Pressure Product | | | | | |
|---|---|---|---|---|---|
| | Resting | 7897±1709 | 7607±1471 | 7910±1577 | NS |
| | Stage 1 | 13349±2997 | 13114±2617 | 12756±2798 | NS |
| | Peak Exercise | 18210±4104 | 17484±3655 | 18842±3791 | 0.008 |

| | | | | | |
|---|---|---|---|---|---|
| HR = Heart Rate sBP = Systolic Blood Pressure dBP = Diastolic Blood Pressure | | | | | |

### 4. Summary

This Example investigated the possibility that xanthine oxidase inhibitors could be used as new anti-ischaemic agents in the treatment of angina pectoris.

Sixty-five patients with stable chronic angina pectoris were recruited for a double blind, randomised, placebo controlled crossover study. The patients were randomised to receive either allopurinol (up to 600 mg/day) or placebo for 6 weeks and then crossed over. Prior to randomisation and at the end of each treatment period, patients underwent an exercise tolerance test.

The Example demonstrates that allopurinol increased median total exercise time to 393 secs (Interquartile range IQR 280-519) from a baseline value of 301 secs (IQR 251-447) and a value on placebo of 307 secs (IQR 232-430) (p <0.001). Allopurinol increased the median time to ST depression to 298 secs (IQR 211-408) from a baseline value of 232 secs (IQR 182-380) and a placebo value of 249 secs (IQR 200-375) (p <0.001).

Therefore, in stable angina pectoris, treatment with a xanthine oxidase inhibitor significantly prolonged total exercise time; the time to ST depression; and the time to exercise induced chest pain.

Although particular embodiments of the invention have been disclosed herein in detail, this has been done by way of example and for the purposes of illustration only. The aforementioned embodiments are not intended to be limiting with respect to the scope of the appended claims, which follow. It is contemplated by the inventors that various substitutions, alterations, and modifications may be made to the invention without departing from the scope of the invention, which is defined by the claims.

### REFERENCES:

1. Knight CJ, Fox KM. Amlodipine versus diltiazem as additional antianginal treatment to atenolol. Centralised European Studies in Angina Research (CESAR) Investigators. Am J Cardiol 1998; 81(2):133-6.
2. Dunselman PH, van Kempen LH, Bouwens LH, Holwerda KJ, Herweijer AH, Bemink PJ. Value of the addition of amlodipine to atenolol in patients with angina pectoris despite adequate beta blockade. Am J Cardiol 1998; 81(2):128-32.
3. Halcox JP, Nour KR, Zalos G, et al. The effect of sildenafil on human vascular function, platelet activation, and myocardial ischemia. J Am Coll Cardiol 2002; 40(7):1232-40.
4. Fox KM, Thadani U, Ma PT, et al. Sildenafil citrate does not reduce exercise tolerance in men with erectile dysfunction and chronic stable angina. Eur Heart J 2003; 24(24):2206-12.
5. Thadani U, Smith W, Nash S, et al. The effect of vardenafil, a potent and highly selective phosphodiesterase-5 inhibitor for the treatment of erectile dysfunction, on the cardiovascular response to exercise in patients with coronary artery disease. J Am Coll Cardiol 2002; 40(11):2006-12.
6. Rousseau MF, Pouleur H, Cocco G, Wolff AA. Comparative efficacy of ranolazine versus atenolol for chronic angina pectoris. Am J Cardiol 2005; 95(3):311-6.
7. Tardif JC, Ponikowski P, Kahan T. Efficacy of the I(f) current inhibitor ivabradine in patients with chronic stable angina receiving beta-blocker therapy: a 4-month, randomized, placebo-controlled trial. Eur Heart J 2009; 30(5):540-8.
8. Saavedra WF, Paolocci N, St John ME, et al. Imbalance between xanthine oxidase and nitric oxide synthase signaling pathways underlies mechanoenergetic uncoupling in the failing heart. Circ Res 2002; 90(3):297-304.
9. Fraker TD, Jr., Fihn SD, Gibbons RJ, et al. 2007 chronic angina focused update of the ACC/AHA 2002 guidelines for the management of patients with chronic stable angina: a report of the American College of Cardiology/American Heart Association Task Force on Practice Guidelines Writing Group to develop the focused update of the 2002 guidelines for the management of patients with chronic stable angina. J Am Coll Cardiol 2007; 50(23):2264-74.

## Claims

1. A xanthine oxidase inhibitor for use in the treatment of one or more symptoms of stable angina pectoris; and wherein the symptom is pain.

2. The xanthine oxidase inhibitor for use according to Claim 1, wherein the treatment is for the prevention, reduction or alleviation, or the delay to onset of the one or more symptoms.

3. The xanthine oxidase inhibitor for use according to Claim 1 or Claim 2, wherein the pain is selection from one or more of chest pain, neck pain and arm pain.

4. The xanthine oxidase inhibitor for use according to any preceding claim, which is selected from one or more of allopurinol, oxypurinol, febuxostat and carprofen or a pharmaceutically acceptable salt or solvate thereof; preferably wherein the inhibitor is allopurinol.

5. The xanthine oxidase inhibitor for use according to any preceding claim, wherein the use is in combination with one or more additional therapeutic agents; preferably wherein the one or more additional therapeutic agents is selected from the group consisting of: statins, for example, simvastatin, atorvastatin, fluvastatin, lovastatin, pravastatin; aspirin; ACE inhibitors; beta- blockers, for example, carvedilol, propranolol, atenolol; nitrates, for example, nitroglycerine, isosorbide mononitrate; calcium-channel blockers, for example, nifedipine, amlodipine; nicorandil; ivabradine; and ranolazine.

6. The xanthine oxidase inhibitor for use according to Claim 5, wherein the one or more additional therapeutic agents is an anti-anginal agent selected from the group consisting of: nitrates; beta-blockers; calcium- channel blockers; nicorandil; ivabradine and ranolazine.

7. The xanthine oxidase inhibitor for use according to any preceding claim, wherein the use comprises the separate, simultaneous or sequential administration of the xanthine oxidase inhibitor with:
(i) a beta-blocker;
(ii) a nitrate;
(iii) nicorandil;
(iv) a calcium-channel blocker;
(v) a beta-blocker and a nitrate;
(vi) a beta-blocker and nicorandil; and/or
(vii) a beta-blocker and a calcium-channel blocker.

8. The xanthine oxidase inhibitor for use according to any preceding claim, wherein the amount of inhibitor is from 300 mg to 900 mg per day; preferably wherein the amount of inhibitor is approximately 600 mg per day, for example, 300 mg BID.

9. The xanthine oxidase inhibitor for use according to any preceding claim, wherein the use comprises administering the inhibitor at an amount of: 100 mg per day for a first week; 300 mg per day for a second week; and 600 mg (e.g. 300 mg BID) for at least a further 4 weeks.

10. A pharmaceutical composition comprising a xanthine oxidase inhibitor and a pharmaceutically acceptable diluent or carrier for use in treating one or more symptoms of stable angina pectoris; and wherein the symptom is pain.

11. The pharmaceutical composition of Claim 10, for the use of claim 10, formulated for modified- or controlled-release of the xanthine oxidase inhibitor; optionally wherein the pharmaceutical composition: (i) is formulated for sustained-release of the xanthine oxidase inhibitor over a time period of at least 8 hours; or (ii) is formulated for sustained-release of the xanthine oxidase inhibitor over a time period of approximately 24 hours.

12. The pharmaceutical composition of Claim 11 or Claim 12, for the use of claim 10, wherein the xanthine oxidase inhibitor is allopurinol or a pharmaceutically acceptable salt or solvate thereof.

13. The pharmaceutical composition of any of Claims 10 to 12, for the use of claim 10, wherein the xanthine oxidase inhibitor and/or the use is as defined in any of Claims 1 to 9.

14. The pharmaceutical composition of any of Claims 10 to 13, for the use of claim 10, which is in the form of a pill, tablet, lacquered tablet, sugar-coated tablet, granule, hard or soft gelatin capsule, aqueous, alcoholic or oily solution, sprinkle formulation, syrup, emulsion or suspension, suppository, solution for injection or infusion, ointment, tincture, spray, transdermal therapeutic system, nasal spray, sublingual spray, aerosol mixture, microcapsule, implant or rod; preferably which is suitable for oral administration, such as a tablet, pill, capsule or spray.

15. A kit comprising a xanthine oxidase inhibitor for use according to any of Claims 1 to 9, or a pharmaceutical composition of any of Claims 10 to 14, for the use of claim 10, and at least one additional therapeutic agent selected from statins, for example, simvastatin, atorvastatin, fluvastatin, lovastatin, pravastatin; aspirin; ACE inhibitors; beta-blockers, for example, carvedilol, propranolol, atenolol; nitrates, for example, nitroglycerine, isosorbide mononitrate; calcium-channel blockers, for example, nifedipine, amlodipine; nicorandil; ivabradine; and ranolazine; and instructions for use in treating angina.

## Patentansprüche

1. Xanthinoxidasehemmer zur Verwendung bei der Behandlung von einem oder mehreren Symptom(en) von stabiler Angina pectoris; und wobei das Symptom Schmerz ist.

2. Xanthinoxidasehemmer zur Verwendung nach Anspruch 1, wobei die Behandlung zur Prävention, Verringerung oder Linderung oder für verzögertes Auftreten von einem oder mehreren Symptom(en) erfolgt.

3. Xanthinoxidasehemmer zur Verwendung nach Anspruch 1 oder 2, wobei der Schmerz ausgewählt ist aus einem oder mehreren Schmerz(en) in Brust, Nacken und Armen.

4. Xanthinoxidasehemmer zur Verwendung nach einem der vorhergehenden Ansprüche, welcher ausgewählt ist aus einem oder mehreren von Allopurinol, Oxypurinol, Febuxostat und Carprofen oder einem pharmazeutisch akzeptablen Salz oder Solvat davon; wobei der Hemmstoff vorzugsweise Allopurinol ist.

5. Xanthinoxidasehemmer zur Verwendung nach einem der vorhergehenden Ansprüche, wobei die Verwendung in Kombination mit einem oder mehreren zusätzlichen therapeutischen Mittel(n) erfolgt; wobei das eine oder die mehreren zusätzlichen therapeutischen Mittel vorzugsweise aus der Gruppe ausgewählt ist/sind, bestehend aus: Statinen, zum Beispiel Simvastatin, Atorvastatin, Fluvastatin, Lovastatin, Pravastatin; Aspirin; ACE-Hemmern; Betablockern, zum Beispiel Carvedilol, Propranolol, Atenolol; Nitraten, zum Beispiel Nitroglycerin; Isosorbidmononitrat; Kalziumkanalblockern, zum Beispiel Nifedipin, Amlodipin; Nicorandil; Ivabradin; und Ranolazin.

6. Xanthinoxidasehemmer zur Verwendung nach Anspruch 5, wobei das eine oder die mehreren zusätzlichen therapeutischen Mittel ein anti-anginöses Mittel ist, das aus der Gruppe ausgewählt ist, bestehend aus: Nitraten; Betablockern; Kalziumkanalblockern; Nicorandil; Livabradin und Ranolazin.

7. Xanthinoxidasehemmer zur Verwendung nach einem der vorhergehenden Ansprüche, wobei die Verwendung die getrennte, gleichzeitige oder aufeinander folgende Verabreichung des Xanthinoxidasehemmers umfasst mit:
(i) einem Betablocker;
(ii) einem Nitrat;
(iii) Nicorandil;
(iv) einem Kalziumkanalblocker;
(v) einem Betablocker und einem Nitrat;
(vi) einem Betablocker und Nicorandil, und/oder
(vii) einem Betablocker und einem Kalziumkanalblocker.

8. Xanthinoxidasehemmer zur Verwendung nach einem der vorhergehenden Ansprüche, wobei die Menge des Hemmstoffes von 300 mg bis 900 mg pro Tag beträgt; wobei die Hemmstoffmenge vorzugsweise bei etwa 600 mg pro Tag liegt, zum Beispiel bei zweimal täglich 300 mg.

9. Xanthinoxidasehemmer zur Verwendung nach einem der vorhergehenden Ansprüche, wobei die Verwendung die Verabreichung des Hemmstoffes in einer Menge von: 100 mg pro Tag für eine erste Woche, 300 mg pro Tag für eine zweite Woche und 600 mg (z. B. 300 mg zweimal täglich) für mindestens vier weitere Wochen umfasst.

10. Pharmazeutische Zusammensetzung, die einen Xanthinoxidasehemmer und einen pharmazeutisch akzeptablen Verdünner oder Trägerstoff umfasst, zur Verwendung bei der Behandlung von einem oder mehreren Symptom(en) von stabiler Angina pectoris; und wobei das Symptom Schmerz ist.

11. Pharmazeutische Zusammensetzung nach Anspruch 10 zur Verwendung nach Anspruch 10, die für die modifizierte oder kontrollierte Freisetzung des Xanthinoxidasehemmers formuliert ist, wobei die pharmazeutische Zusammensetzung optional: (i) für die anhaltende Freisetzung des Xanthinoxidasehemmers über einen Zeitraum von mindestens 8 Stunden formuliert ist; oder (ii) für die anhaltende Freisetzung des Xanthinoxidasehemmers über einen Zeitraum von etwa 24 Stunden formuliert ist.

12. Pharmazeutische Zusammensetzung nach Anspruch 11 oder Anspruch 12 zur Verwendung nach Anspruch 10, wobei der Xanthinoxidasehemmer Allopurinol oder ein pharmazeutisch akzeptables Salz oder Solvat davon ist.

13. Pharmazeutische Zusammensetzung nach einem der Ansprüche 10 bis 12 zur Verwendung nach Anspruch 10, wobei der Xanthinoxidasehemmer und/oder die Verwendung wie in einem der Ansprüche 1 bis 9 definiert ist.

14. Pharmazeutische Zusammensetzung nach einem der Ansprüche 10 bis 13 zur Verwendung nach Anspruch 10, welche in der Form einer Pille, einer Tablette, einer Filmtablette, einer zuckerüberzogenen Tablette, eines Granulats, einer Hart- oder Weichgelatinekapsel, einer wässrigen, alkoholischen oder öligen Lösung, einer Sprühformulierung, eines Sirups, einer Emulsion oder Suspension, eines Zäpfchens, einer Lösung zur Injektion oder Infusion, einer Salbe, einer Tinktur, eines Sprays, eines transdermalen therapeutischen Systems, eines Nasensprays, eines Sublingualsprays, einer Aerosolmischung, einer Mikrokapsel, eines Implantats oder Stäbchens vorliegt; welche vorzugsweise zur oralen Verabreichung geeignet ist, wie beispielsweise eine Tablette, Pille, Kapsel oder ein Spray.

15. Kit, das einen Xanthinoxidasehemmer zur Verwendung nach einem der Ansprüche 1 bis 9, oder eine pharmazeutische Zusammensetzung nach einem der Ansprüche 10 bis 14 zur Verwendung nach Anspruch 10 und mindestens ein zusätzliches therapeutisches Mittel, ausgewählt aus Statinen, zum Beispiel Simvastatin, Atorvastatin, Fluvastatin, Lovastatin, Pravastatin; Aspirin; ACE-Hemmern; Betablockern, zum Beispiel Carvedilol, Propranolol, Atenolol; Nitraten, zum Beispiel Nitroglycerin, Isosorbidmononitrat; Kalziumkanalblockern, zum Beispiel Nifedipin, Amlodipin; Nicorandil; Ivabradin; und Ranolazin sowie Anweisungen zur Verwendung bei der Behandlung von Angina, umfasst.

## Revendications

1. Inhibiteur de la xanthine-oxydase à utiliser dans le traitement de l'un ou de plusieurs symptômes de l'angine de poitrine stable ; et dans laquelle le symptôme est la douleur.

2. Inhibiteur de la xanthine-oxydase à utiliser selon la revendication 1, dans laquelle le traitement est pour la prévention, la réduction ou le soulagement, ou le retard d'apparition de l'un ou de plusieurs symptômes.

3. Inhibiteur de la xanthine-oxydase à utiliser selon la revendication 1 ou la revendication 2, dans lequel la douleur est sélectionnée parmi le groupe constitué de la douleur dans la poitrine, la douleur dans le cou et la douleur dans le bras.

4. Inhibiteur de la xanthine-oxydase à utiliser selon l'une quelconque des revendications précédentes, qui est sélectionné parmi une ou plusieurs des substances suivantes : allopurinol, oxypurinol, fébuxostat et carprofène ou un sel ou solvate pharmaceutiquement acceptable de ces dernières ; de préférence, dans laquelle l'inhibiteur est l'allopurinol.

5. Inhibiteur de la xanthine-oxydase à utiliser selon l'une quelconque des revendications précédentes, dans lequel l'utilisation est en combinaison avec l'un ou plusieurs agent(s) thérapeutique(s) supplémentaire(s) ; de préférence dans laquelle le un ou les plusieurs agent(s) thérapeutique(s) supplémentaire(s) est(sont) sélectionné(s) parmi le groupe constitué de : statines, par exemple simvastatine, atorvastatine, fluvastatine, lovastatine, pravastatine ; aspirine, inhibiteurs de l'ECA ; bêta-bloquants, par exemple carvédilol, propranolol, aténolol ; nitrates, par exemple nitroglycérine, mononitrate d'isosorbide ; inhibiteurs calciques, par exemple nifédipine, amiodipine ; nicorandil ; ivabradine ; et ranolazine.

6. Inhibiteur de la xanthine-oxydase à utiliser selon la revendication 5, dans laquelle le ou les plusieurs agent(s) thérapeutique(s) supplémentaire(s) est un agent anti-angineux sélectionné parmi le groupe constitué de : nitrates ; béta-bloquants ; inhibiteurs calciques ; nicorandil ; ivabradine et ranolazine.

7. Inhibiteur de la xanthine-oxydase à utiliser selon l'une quelconque des revendications précédentes, dans laquelle l'utilisation comprend l'administration séparée, simultanée ou séquentielle de l'inhibiteur de la xanthine-oxydase avec :
(i) un bêta-bloquant;
(ii) un nitrate ;
(iii) le nicorandil ;
(iv) un inhibiteur calcique :
(v) un bêta-bloquant et un nitrate ;
(vi) un bêta-bloquant et le nicorandil ; et/ou
(vii) un bêta-bloquant et un inhibiteur calcique.

8. Inhibiteur de la xanthine-oxydase à utiliser selon l'une quelconque des revendications précédentes, dans laquelle la quantité d'inhibiteur se situe entre 300 mg et 900 mg par jour ; de préférence dans laquelle la quantité d'inhibiteur est approximativement 600 mg par jour, par exemple, 300 mg deux fois par jour.

9. Inhibiteur de la xanthine-oxydase à utiliser selon l'une quelconque des revendications précédentes, dans laquelle l'utilisation comprend l'administration d'un inhibiteur en une quantité de : 100 mg par jour pour une première semaine ; 300 mg par jour pour une seconde semaine ; et 600 mg (par exemple 300 mg deux fois par jour) pour au moins 4 semaines supplémentaires.

10. Composition pharmaceutique comprenant un inhibiteur de la xanthine-oxydase et un diluant ou un vecteur pharmaceutiquement acceptable à utiliser pour traiter l'un ou plusieurs symptôme(s) de l'angine de poitrine stable ; et dans laquelle le symptôme est la douleur.

11. Composition pharmaceutique selon la revendication 10, pour l'utilisation de la revendication 10, formulée pour la libération modifiée ou contrôlée de l'inhibiteur de la xanthine-oxydase ; optionnellement, dans laquelle la composition pharmaceutique : (i) est formulée pour la libération prolongée de l'inhibiteur de la xanthine-oxydase sur une période d'au moins 8 heures ; ou (ii) est formulée pour la libération prolongée de l'inhibiteur de la xanthine-oxydase sur une période d'approximativement 24 heures.

12. Composition pharmaceutique selon la revendication 11 ou la revendication 12, pour l'utilisation de la revendication 10, dans laquelle l'inhibiteur de la xanthine-oxydase est l'allopurinol ou un sel ou solvate pharmaceutiquement acceptable de ce dernier.

13. Composition pharmaceutique selon l'une quelconque des revendications 10 à 12, pour l'utilisation de la revendication 10, dans lequel l'inhibiteur de la xanthine-oxydase et/ou l'utilisation est telle que définie dans l'une quelconque des revendications 1 à 9.

14. Composition pharmaceutique selon l'une quelconque des revendications 10 à 13, pour l'utilisation de la revendication 10, qui se présente sous la forme d'une pilule, d'un comprimé, d'un comprimé laqué, d'un comprimé pelliculé, d'un granule, d'une capsule ou d'une gélule, d'une solution aqueuse, alcoolisée ou huileuse, d'une formulation à verser en pluie, d'un sirop, d'une émulsion ou d'une suspension, d'un suppositoire, d'une solution pour injection ou perfusion, d'une pommade, d'une teinture, d'un vaporisateur, d'un système transdermique thérapeutique, d'un vaporisateur nasal, d'un vaporisateur sublingual, d'un mélange en aérosol, d'une microcapsule, d'un implant ou d'une tige ; de préférence, qui convient pour l'administration par voie orale, telle qu'un comprimé, une pilule, une capsule ou un vaporisateur.

15. Kit comprenant un inhibiteur de la xanthine-oxydase à utiliser selon l'une quelconque des revendications 1 à 9, ou une composition pharmaceutique selon l'une quelconque des revendications 10 à 14, pour l'utilisation de la revendication 10, et au moins un agent thérapeutique supplémentaire sélectionné parmi des statines, par exemple simvastatine, atorvastatine, fluvastatine, lovastatine, pravastatine, aspirine ; des inhibiteurs de l'ECA ; des bêta-bloquants, par exemple carvédilol, propranolol, aténolol ; des nitrates, par exemple nitroglycérine, mononitrate d'isosorbide ; des inhibiteurs calciques, par exemple nifédipine, amiodipine ; le nicorandil ; l'ivabradine ; et la ranolazine ; et le mode d'emploi pour traiter l'angine.
